# EUROPÄISCHE PATENTSCHRIFT

(11) **EP 0 289 976 B1**
(45) Veröffentlichungstag und Bekanntmachung des Hinweises auf die Patenterteilung: **11.08.1993**
(21) Anmeldenummer: 88107019.7
(22) Anmeldetag: 02.05.1988
(51) Int. Cl.: G01N 21/64, G01N 33/18, C12Q 1/18

(54) **Verfahren und Einrichtung zum Toxizitätsnachweis in Oberflächengewässern sowie in Trink- und Brauchwasser**
Method and device for detecting the toxity of superficial waters as well as of drinking or nondrinking water
Procédé et dispositif pour détecter la toxicité d'eaux superficielles ainsi que de l'eau potable ou non potable

(30) Priorität: 06.05.1987 DE 3715114
(43) Veröffentlichungstag der Anmeldung: 09.11.1988
(73) Patentinhaber: Krause, Hans, Dr., D-61231 Bad Nauheim (DE); Maske, Helmut, Dr., D-24116 Kiel (DE)
(72) Erfinder: Krause, Hans, Dr., D-61231 Bad Nauheim (DE); Maske, Helmut, Dr., D-24116 Kiel (DE)
(74) Vertreter: Kraus, Walter, Dr.

(56) Entgegenhaltungen:
- DE-A- 3 412 023
- PHOTOSYNTHETIA Band 18, 1984, Seiten 117-127; M. HAVAUX et al.: "Effects of chilling temperatures on prompt and delayed chlorophyll fluorescence in maize and barley leaves"
- BULL. ENVIRONM. CONTAM. TOXICOL. Band 28, 1982, Seiten 385-395; G. BENECKE et al.: "Use of Algal Fluorescence for an Automated Biological Monitoring System"
- SOVIET INVENTIONS ILLUSTRATED Sektion Chemie, Woche K 25, 10. August 1983, Zusammenfassung Nr. 63133 K/26, D15T06, Derwent Publications Ltd., London, GB & SU-A-95 0682

## Beschreibung

Die Erfindung betrifft ein Verfahren zum Toxizitätsnachweis in Oberflächengewässern sowie in Trink- und Brauchwasser, bei dem die Fluoreszenz einer Wasserprobe gemessen wird. Außerdem betrifft die Erfindung eine Einrichtung zum Toxizitätsnachweis in Oberflächengewässern sowie in Trink- und Brauchwasser, umfassend eine Fluoreszenzmeßeinrichtung für Fluoreszenzmessungen an Flüssigkeiten.

Wie dem Abschnitt "Wasseruntersuchungen mit Hilfe von Toxizitätstests" von W. K. Besch in dem Buch "Limnologie für die Praxis", Herausgeber Besch, Hamm, Lenhardt, Melzer, Scharf, Steinberg, Landsberg/Lech 1984 zu entnehmen ist, basieren die klassischen Ansätze von Toxizitätstest in Fließgewässern auf physikalisch-chemischen Meßmethoden oder Bioassays mit gezüchteten oder gehälterten Organismen, wie zum Beispiel mit Algen, Bakterien, Krebsen, Insekten oder Fischen. Jede dieser klassischen Meßmethoden hat den Nachteil, daß sie einen großen technischen und/oder zeitintensiven Aufwand erfordert. Außerdem sind Toxizitätsaussagen, die aus Bioassays gewonnen werden, erst nach mehreren Stunden oder sogar erst nach ein oder zwei Tagen möglich, wie in dem Buch "Gewässer- und Pflanzenschutz" von F. Meinck aus der Schriftenreihe des Vereins für Wasser-, Boden- und Lufthygiene, Band 37, Gustav Fischer Verlag, Stuttgart 1972 und in dem Abschnitt "Toxizitätstest mit Goldorfe und Zebraberbling" von W.K. Besch, B.W. Scharf und E. Mayer des Buches "Wassergefährdende Stoffe" von L. Roth (Herausgeber), ecomed, Landsberg/Lech, 1985 angegeben ist.

Eine neuere Methode der Erfassung von Herbiziden in Fließgewässern, die in der Veröffentlichung "Use of Algal Fluorescence for an Automated Biological Monitoring System" von G. Benecke, W. Falke und W. Schmidt in Bull. Environm. Contam. Toxicol. 28, 1982, Seite 385 bis 395 beschrieben ist, beruht auf der Analyse von Fluoreszenzinduktionskurven an Standard-Algenkulturen, und zwar darauf, daß die prompte Fluoreszenz durch Vergiftung mit Herbiziden insgesamt etwa um den Faktor zwei zunimmt. Die hierbei erforderliche Analyse der prompten Fluoreszenz-Induktionskurven erfordert einen großen rechnerischen Aufwand, und trotzdem ist die Interpretation der Analyseergebnisse nicht eindeutig und hängt von experimentellen Randbedingungen ab, insbesondere von der Temperatur, der Licht-Vorbehandlung und dem allgemeinen physiologischen Zustand der Algen.

Auf der Analyse der Fluoreszenzinduktion beruhen auch das Verfahren und die Vorrichtung, welche in der DE-OS 34 12 023 beschrieben sind und zur Schnellbestimmung von Schadstoffen in Gewässern mittels eines Biomaterials mit Fähigkeit zu photosynthetischen Primärprozessen dienen. Hier werden als Biomaterial Thylakoide von unter standardisierten Bedingungen gezogenen höheren Pflanzen verwendet, deren Zellhomogenate durch Zentrifugation aufgetrennt und gefriergetrocknet sind, und das Biomaterial wird in Chargen gleicher Grundaktivität zusammen mit anorganischen und/oder organischen Stabilisatoren als Suspension in standardisierten Proben eingesetzt. Die Schadstoffbestimmung erfolgt in einem Meßmedium durch Messen der Fluoreszenzinduktion zu zwei in ihrem Abstand festgelegten Zeitpunkten einer Zeitspanne der Belichtung der das Biomaterial enthaltenden standardisierten Probe und durch einen Vergleich der Meßwertdifferenz mit Solldifferenzwerten. Diese Analysemethode erfordert die sehr aufwendige Herstellung einer Zellhomogenatprobe, die auf Sauerstoffentwicklung und Fluoreszenzinduktion untersucht wird, wobei nur die prompte Fluoreszenz gemessen wird. Gegenüber der weiter oben genannten Analyse von Fluoreszenzinduktionskurven an Standard-Algenkulturen bedeutet das in nachteiliger Weise eine noch stärkere Spezialisierung hinsichtlich der Probensubstanz, da hier nicht generell Algenkulturen verwendet werden, sondern spezielle Zellhomogenate aus Thylakoiden höherer Pflanzen. Zwar wird gegenüber der weiter oben genannten Analyse von Fluoreszenzinduktionskurven durch das Verfahren nach der DE-OS 34 12 023 der rechnerische Aufwand dadurch erheblich vermindert, daß nur zwei Meßpunkte aus der Fluoreszenzinduktionskurve herausgegriffen werden können. Jedoch wird hierdurch die Aussagefähigkeit des Verfahrens ganz erheblich eingeschränkt, so daß das Verfahren nur ein grobes Verfahren ist. Außerdem ist keine kontinuierliche Messung möglich.

Weiterhin hat L.O. Björn in der Zeitschrift "Photochem. Photobiol." 1971, 13, Seite 5 bis 20 Untersuchungen über die Abklingkinetik der verzögerten Fluoreszenz in Abhängigkeit von einer Herbizideinwirkung veröffentlicht. Auch in diesem Falle ist die Interpretation der erhaltenen Ergebnisse nicht eindeutig. Zwar nimmt die verzögerte Fluoreszenz bei Schädigung bzw. Zerstörung der Photosynthese ab, jedoch hängt die relative Änderung von dem Zeitfenster der verzögerten Fluoreszenzmessung ab, in welchem nach dem Abschalten der Anregung gemessen wird, wie aus der Dissertation "Verzögerte Fluoreszenz photoautotropher Algen" von H. Krause, Universität Regensburg, 1986 zu entnehmen ist.

Aufgabe der vorliegenden Erfindung ist es insbesondere, Herbizide und andere toxische Stoffe in Gewässern schnell, kontinuierlich und mit großer zeitlicher Auflösung bei relativ geringem technischem Aufwand in ihrer toxischen Wirkung nachzuweisen bzw. zu ermitteln.

Diese Aufgabe wird mit einem Verfahren der eingangs genannten Art erfindungsgemäß dadurch gelöst, daß die Korrelation zwischen der prompten Fluoreszenz und der verzögerten Fluoreszenz einer Wasserprobe bestimmt wird, die einen toxizitätsempfindlichen Bioorganismus enthält.

Außerdem wird diese Aufgabe gelöst durch eine Einrichtung der eingangs genannten Art, die eine Fluoreszenzmeßeinrichtung für Fluoreszenzmessungen an Flüssigkeiten aufweist und sich erfindungsgemäß dadurch auszeichnet, daß die Fluoreszenzmeßeinrichtung eine Kombination aus einer ersten Fluoreszenzmeßeinrichtung zum Messen der prompten Fluoreszenz und einer zweiten Fluoreszenzmeßeinrichtung zum Messen der verzögerten Fluoreszenz umfaßt und den Ausgängen oder dem gemeinsamen Ausgang der beiden Fluoreszenzeinrichtungen eine Signalverknüpfungs- bzw. -korrelierungseinrichtung zum Miteinanderverknüpfen bzw. Korrelieren ihrer Ausgangssignale nachgeschaltet ist.

Es wurde nämlich, obwohl, wie oben dargelegt, weder die Analyseergebnisse der prompten Fluoreszenz eine eindeutige Aussage über die Toxizität ermöglichen, noch die Analyse der verzögerten Fluoreszenz eine eindeutige Interpretation der Toxizität gestattet, im Rahmen der vorliegenden Erfindung überraschenderweise gefunden, daß der Korrelationswert, insbesondere das Verhältnis, zwischen der prompten Fluoreszenz und der verzögerten Fluoreszenz einer Wasserprobe ein eindeutiges Maß für die Toxizität dieser Wasserprobe ist.

Eingehende Untersuchungen der Erfinder, die im Rahmen der vorliegenden Erfindung durchgeführt wurden, haben gezeigt, daß die prompte und verzögerte Fluoreszenz im Tagesgang aufgrund der Lichtadaption zwar stark schwanken, jedoch diese Schwankung ziemlich synchron und in etwa gleicher relativer Amplitude erfolgt, so daß sich die Korrelation, insbesondere das Verhältnis, des Werts, insbesondere der Intensität, der prompten Fluoreszenz zu dem Wert, insbesondere der Intensität, der verzögerten Fluoreszenz kaum ändert. Änderungen in der Korrelation, insbesondere im Verhältnis, beider Fluoreszenzwerte, nämlich des Werts der prompten Fluoreszenz und des Werts der verzögerten Fluoreszenz, zueinander können daher als Störungen der Zellphysiologie interpretiert werden, d.h. im Normalfall als Vergiftungen.

Der vorliegenden Erfindung liegt daher, ausgehend von der obigen überraschenden Feststellung der Erfinder, der Vorschlag zugrunde, durch die gleichzeitige Messung der prompten und der verzögerten Fluoreszenz, d.h. insbesondere von deren Intensität, in geeigneten Zeitbereichen mit einem oder mehreren Meßpunkten, und durch Vergleich der Fluoreszenzintensitäten der Meßpunkte, insbesondere durch einen Algorithmus, auf die Wirkung von toxischen Wasserinhaltsstoffen auf einen im Gewässer befindlichen Bioorganismus, insbesondere auf einen im Gewässer natürlich vorkommenden Bioorganismus, vorzugsweise Phytoplankton, zu schließen.

Zur technischen Durchführung dieses erfindungsgemäßen Toxizitätsnachweises können sowohl die räumliche Trennung, beispielsweise durch Verwendung eines kontinuierlichen Strömungssystems, wie auch die zeitliche Trennung, beispielsweise durch Verwendung der Lichtimpulsmethode, verwendet werden.

Die Erfindung schlägt also vor, die beiden Fluoreszenzkomponenten, insbesondere die Intensität der prompten Fluoreszenz und die Intensität der verzögerten Fluoreszenz, in Relation zueinander zu bewerten, da ihre Signalstärken bei Vergiftung gegensätzliche Tendenzen zeigen. Jedes einzelne Signal zeigt zwar relative Schwankungen in Abhängigkeit von der Beleuchtungsvorgeschichte, wie sie beispielsweise durch den Verlauf des Tageslichts gegeben ist, jedoch ist der Korrelationswert, insbesondere das Verhältnis, beider Signale zueinander in etwa konstant.

Im einzelnen zeichnet sich das erfindungsgemäße Verfahren dadurch aus, daß die Korrelation zwischen der prompten Fluoreszenz und der verzögerten Fluoreszenz einer Wasserprobe, die einen toxizitätsempfindlichen Bioorganismus enthält, bestimmt wird und mit der entsprechenden Korrelation verglichen wird, die an einer nichtvergifteten Probe bestimmt worden ist.

Insbesondere kann das Verfahren so ausgeführt werden, daß die Korrelation innerhalb eines vorbestimmten Zeitfensters ab dem Ende des Einwirkens eines Anregungslichts für die verzögerte Fluoreszenz auf die Probe gemessen wird, wobei dieses Zeitfenster vorzugsweise eine Breite von 0,1 bis 10 Sekunden hat und bevorzugt in einem Bereich von 0,5 bis 500 Sekunden nach dem Ende des Einwirkens des Anregungslichts für die verzögerte Fluoreszenz liegt.

Brauch- und Trinkwasser ohne geeigneten natürlichen Bioorganismusbestand, insbesondere ohne natürlichen Phytoplanktonbestand, können trotzdem auf toxische Inhaltsstoffe untersucht werden, indem die Wasserprobe beispielsweise mit einer Kulturlösung eines toxizitätsempfindlichen Bioorganismus, insbesondere mit einer Phytoplankton-Kulturlösung, vermischt wird.

Wenn die entsprechende Korrelation zwischen prompter und verzögerter Fluoreszenz an einer nichtvergifteten Wasserprobe bestimmt wird, kann durch übliche Methoden, wie beispielsweise Massenspektrometrie oder Chromatographie, festgestellt werden, ob die Wasserprobe tatsächlich nicht vergiftet ist. Diese Korrelation kann durch Messungen an einer nichtvergifteten Probe als Standard erstellt und für viel später erfolgende aktuelle Messungen verwendet werden.

Wie die vorstehenden Ausführungen zeigen, hat sich als toxizitätsempfindlicher Bioorganismus mit Vorteil ein Plankton als brauchbar erwiesen, und zwar insbesondere ein Phytoplankton. Als toxizitätsempfindliche Bioorganismen können auch Bakterien oder Dinoflagellanten verwendet werden.

Eine bevorzugte Ausführungsform des erfindungsgemäßen Verfahrens zeichnet sich dadurch aus, daß die prompte und die verzögerte Fluoreszenz, insbesondere deren Intensität, mittels Licht der gleichen Wellenlänge angeregt wird. Das hat den Vorteil, daß man nur eine einzige Lichtquelle zum Anregen der prompten und verzögerten Fluoreszenz benötigt. Vorzugsweise kann, insbesondere bei Verwendung von Phytoplankton als toxizitätsempfindlicher Bioorganismus, zum Anregen der prompten und verzögerten Fluoreszenz Licht im Wellenbereich um 440 nm oder Licht im Wellenbereich um 660 bis 685 nm verwendet werden.

Es ist jedoch auch möglich, die prompte und verzögerte Fluoreszenz mittels Licht unterschiedlicher Wellenlängen anzuregen, wodurch in vorteilhafter Weise eine einfachere störungsfreie Trennung der beiden Fluoreszenzmessungen, nämlich der Messung der prompten und der verzögerten Fluoreszenz, ermöglicht wird. Vorzugsweise wird hierbei, insbesondere bei Verwendung von Phytoplankton als toxizitätsempfindlicher Bioorganismus, die prompte Fluoreszenz mittels Licht im Wellenlängenbereich um 440 nm und/oder um 660 bis 685 nm und die verzögerte Fluoreszenz mittels Licht im Wellenlängenbereich um 700 bis 730 nm angeregt.

Hinsichtlich der Messung der Fluoreszenz, insbesondere der Intensität der Fluoreszenzstrahlung, ist es sowohl möglich, die prompte und die verzögerte Fluoreszenz im gleichen Wellenlängenbereich zu messen, als auch die prompte und verzögerte Fluoreszenz in unterschiedlichen Wellenlängenbereichen zu messen, d.h. im letzteren Fall die prompte Fluoreszenz in einem anderen Wellenlängenbereich als die verzögerte Fluoreszenz zu messen. Im ersteren Falle können beispielsweise die prompte und die verzögerte Fluoreszenz beide im Wellenlängenbereich um 685 nm gemessen werden, während im letzteren Fall die Messung so durchgeführt werden kann, daß die prompte Fluoreszenz im Wellenlängenbereich um 730 nm und die verzögerte Fluoreszenz im Wellenlängenbereich um 685 nm gemessen wird.

Die mit der Erfindung zur Verfügung gestellte Einrichtung zum Toxizitätsnachweis in Oberflächengewässern kann entweder als Durchflußmeßeinrichtung im kontinuierlichen Strömungssystem der Wasserprobe ausgebildet sein, wodurch sich eine räumliche Trennung zwischen dem Meßsystem zur Messung der prompten und dem Meßsystem zur Messung der verzögerten Fluoreszenz ergibt, oder die Einrichtung kann so ausgebildet sein, daß sie ein für die Messung der prompten und verzögerten Fluoreszenz gemeinsames Meßsystem besitzt, in welchem die prompte und verzögerte Fluoreszenz nacheinander, beispielsweise durch Lichtimpulse, angeregt und/oder gemessen werden, wodurch sich eine zeitliche Trennung der beiden Fluoreszenzmessungen ergibt.

Im ersteren Falle, also bei räumlicher Trennung der Fluoreszenzmessungen, zeichnet sich die erfindungsgemäße Einrichtung bevorzugt aus durch zwei strömungsmäßig hintereinandergeschaltete Fluoreszenzmeß-Durchflußküvetten, deren Verbindungsleitung eine Lichtfalle enthält und von denen der in Durchflußrichtung ersten Fluoreszenzmeß-Durchflußküvette ein erster Lichtdetektor zum Messen der prompten Fluoreszenz und der in Durchflußrichtung zweiten Fluoreszenzmeß-Durchflußküvette ein zweiter Lichtdetektor zum Messen der verzögerten Fluoreszenz zugeordnet ist.

Hierbei kann die Ausbildung hinsichtlich der Lichtquelle so sein, daß
(a) der ersten Fluoreszenzmeß-Durchflußküvette eine Lichtquelle zum Anregen von prompter und verzögerter Fluoreszenz zugeordnet ist, oder
(b) der ersten Fluoreszenzmeß-Durchflußküvette eine erste Lichtquelle zum Anregen der prompten Fluoreszenz zugeordnet ist und strömungsmäßig zwischen die erste und zweite Fluoreszenzmeß-Durchflußküvette eine Fluoreszenzanregungs-Durchflußküvette geschaltet ist, der eine zweite Lichtquelle zum Anregen von verzögerter Fluoreszenz zugeordnet ist, wobei die Lichtfalle in der Verbindungsleitung zwischen der Fluoreszenzanregungs-Durchflußküvette und der zweiten Fluoreszenzmeß-Durchflußküvette vorgesehen sein kann.

In beiden vorstehenden Ausführungsformen kann in der Auslaßleitung der zweiten Fluoreszenzmeß-Durchflußküvette eine weitere Lichtfalle vorgesehen sein.

Im Falle der zeitlichen Trennung der Fluoreszenzmessungen ist die erfindungsgemäße Einrichtung bevorzugt so ausgebildet, daß sie eine gemeinsame Fluoreszenzmeß-Küvette für die Messung der prompten und verzögerten Fluoreszenz aufweist und diese beiden Fluoreszenzarten zeitlich nacheinander gemessen werden, wobei die gemeinsame Fluoreszenzmeß-Küvette auch eine Durchflußküvette sein kann, die dann jedoch diskontinuierlich im Durchfluß betrieben wird, d.h. in einer ersten Durchflußzeit mit der Probe gefüllt, dann der Durchfluß unterbrochen und die Fluoreszenz gemessen, und danach die Durchflußküvette im Durchfluß von der eben gemessenen Probe entleert und beispielsweise mit einer neuen Probe gefüllt wird.

Die vorstehenden sowie weitere Vorteile und Merkmale der Erfindung seien nachfolgend unter Bezugnahme auf die Figuren 1 bis 7 der Zeichnung anhand einiger, besonders bevorzugter Ausführungsformen des Verfahrens und der Einrichtung nach der Erfindung näher erläutert; es zeigen:
- Figur 1: eine erste Ausführungsform der erfindungsgemäßen Einrichtung, in welcher die Intensität der prompten und verzögerten Fluoreszenz räumlich getrennt voneinander im Durchflußverfahren gemessen werden, wobei die Einrichtung schematisch in Blockdarstellung gezeigt ist;
- Figur 2: eine erste Variante der Anordnung der Fluoreszenzmeß-Durchflußküvetten, der Lichtquelle und der Lichtdetektoren, die in der Einrichtung nach Figur 1 verwendet werden kann;
- Figur 3: eine zweite Variante der Anordnung der Fluoreszenzmeß-Durchflußküvetten, der Lichtquellen und der Lichtdetektoren, die in der Einrichtung nach Figur 1 verwendet werden kann;
- Figur 4: eine Anordnung der Fluoreszenzmeß-Küvette, der Lichtquelle und des Lichtdetektors für eine Ausführungsform der erfindungsgemäßen Einrichtung, in der mit zeitlicher Trennung der Fluoreszenzmessungen gearbeitet wird; und
- Figur 5: eine in nähere Einzelheiten gehende Ausführungsform einer erfindungsgemäßen Einrichtung gemäß Figur 1 mit einer Anordnung der Fluoreszenzmeß-DurchFlußküvetten, der Lichtquelle und der Lichtdetektoren gemäß der Figur 2;
- Figur 6: den linken oberen Teil der Figur 5 in einer ersten abgewandelten Ausführungsform; und
- Figur 7: den linken oberen Teil der Figur 5 in einer zweiten abgewandelten Ausführungsform.

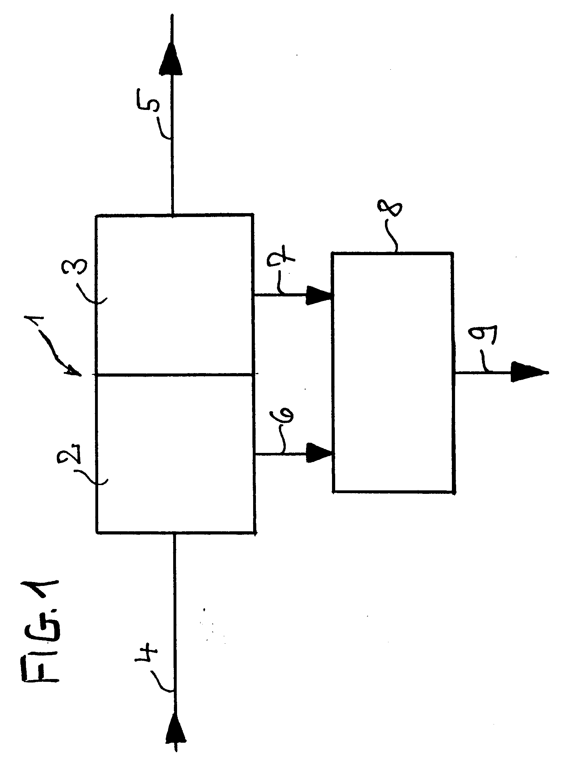

Es sei zunächst auf Figur 1 Bezug genommen, in welcher der grundsätzliche Aufbau einer im kontinuierlichen Strömungssystem betriebenen Einrichtung zum Toxizitätsnachweis in Oberflächengewässern dargestellt ist. Diese Einrichtung umfaßt eine Fluoreszenzmeßeinrichtung 1 für Fluoreszenzmessungen an Flüssigkeiten. Diese Fluoreszenzmeßeinrichtung 1 ist eine Kombination aus einer ersten Fluoreszenzmeßeinrichtung 2 zum Messen der prompten Fluoreszenz und einer zweiten Fluoreszenzmeßeinrichtung 3 zum Messen der verzögerten Fluoreszenz. Diese beiden Fluoreszenzmeßeinrichtungen 2 und 3 werden nacheinander von der Meßprobe durchströmt, wobei die Meßprobenzuführung mit 4 und die Meßprobenabführung mit 5 bezeichnet sind. Der Meßsignalausgang 6 der Fluoreszenzmeßeinrichtung 2 für die prompte Fluoreszenz und der Meßsignalausgang 7 der Fluoreszenzmeßeinrichtung 3 für die verzögerte Fluoreszenz, an denen Meßsignale erscheinen, welche die Intensität der prompten bzw. der verzögerten Fluoreszenz repräsentieren, sind beide mit dem Signaleingang oder den Signaleingängen einer ihnen gemeinsamen Signalverknüpfungs- bzw. -korrelierungseinrichtung 8 verbunden, welche die beiden eingegebenen Meßsignale durch einen Algorithmus miteinander verknüpft bzw. korreliert, so daß am Ausgang 9 dieser Signalverknüpfungs- bzw. -korrelierungseinrichtung 8 eine Ausgangsgröße erhalten wird, welche ein eindeutiges Maß für die Toxizität der Meßprobe ist.

Im einfachsten Falle kann der Algorithmus der Quotient der beiden Meßsignale, die am Meßsignalausgang 6 und 7 erscheinen, sein, so daß am Ausgang 9 das arithmetische Verhältnis dieser beiden Meßsignale als Ausgangswert für die Toxizität erhalten wird.

In der Ausführungsform der Einrichtung, in welcher die Messung der prompten und verzögerten Fluoreszenz in einem gemeinsamen Meßsystem zeitlich nacheinander durchgeführt wird, sind die Fluoreszenzmeßeinrichtung 2 und die Fluoreszenzmeßeinrichtung 3 zu einer einzigen Fluoreszenzmeßeinrichtung kombiniert, die einen gemeinsamen, einzigen Meßsignalausgang hat, an welchem die Meßsignale für die prompte und die verzögerte Fluoreszenz zeitlich nacheinander erhalten werden, wobei jedoch im übrigen die Signalverknüpfungs- bzw. -korrelierungseinrichtung 8 in gleicher Weise, wie oben dargelegt, ausgebildet ist und arbeitet, indem sie die zeitlich nacheinander erhaltenen Meßsignale für die prompte und verzögerte Fluoreszenz genauso miteinander korreliert bzw. verknüpft.

Bei der zuletztgenannten Ausführungsform kann allerdings auch der gemeinsamen Fluoreszenzmeß-Küvette je ein gesonderter Lichtdetektor zum Messen der prompten und zum Messen der verzögerten Fluoreszenz zugeordnet sein, in welchem Falle sich dann hinsichtlich der Meßsignalausgänge 6 und 7 die gleiche Anordnung ergibt, wie sie in Figur 1 dargestellt ist.

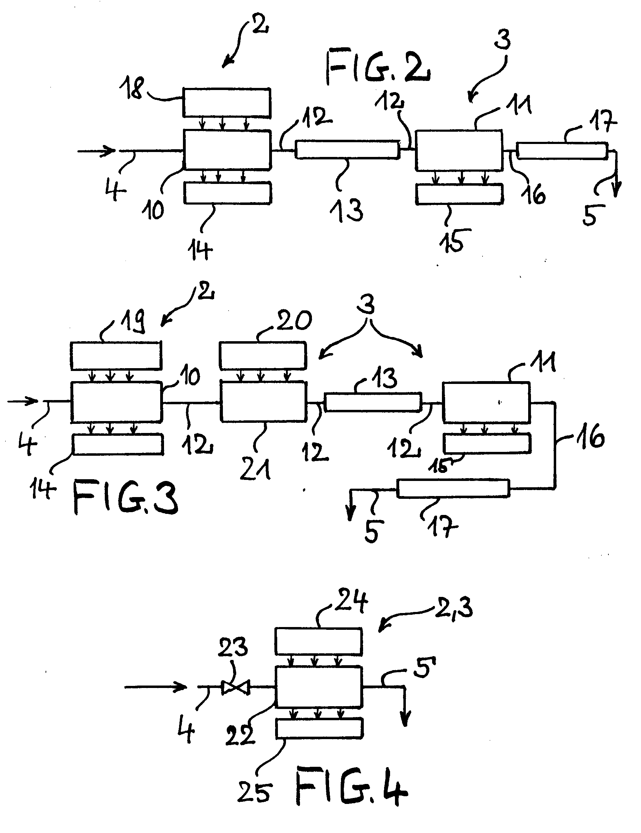

Es sei nun näher auf die Figuren 2 und 3 eingegangen, die zwei unterschiedliche Ausbildungen der Fluoreszenzanregungs- und -meßanordnung zeigen, die beide in der Meßeinrichtung nach Figur 1, sofern diese im kontinuierlichen Probendurchflußverfahren betrieben wird, verwendbar sind, wobei für gleichartige Teile gleiche Bezugszeichen wie in Figur 1 benutzt werden, hinsichtlich deren, um Wiederholungen zu vermeiden, auf die vorstehenden Ausführungen zu Figur 1 verwiesen wird. Sowohl bei der Ausbildung der Einrichtung nach Figur 2 als auch bei der Ausbildung der Einrichtung nach Figur 3 sind zwei strömungsmäßig hintereinander geschaltete Fluoreszenzmeß-Durchflußküvetten 10 und 11 vorgesehen, deren Verbindungsleitung 12 für das Zuführen der Flüssigkeitsprobe von der ersten Fluoreszenzmeß-Durchflußküvette 10 zu der zweiten Fluoreszenzmeß-Durchflußküvette 11 eine Lichtfalle 13 enthält. Der in Durchflußrichtung der Flüssigkeitsprobe ersten Fluoreszenzmeß-Durchflußküvette 10 ist ein erster Lichtdetektor 14 zum Messen der prompten Fluoreszenz zugeordnet, und der in Durchflußrichtung der Flüssigkeitsprobe zweiten Fluoreszenzmeß-Durchflußküvette 11 ist ein zweiter Lichtdetektor 15 zum Messen der verzögerten Fluoreszenz zugeordnet. Schließlich ist in beiden Fällen in der Auslaßleitung 16 der zweiten Fluoreszenzmeß-Durchflußküvette 11, durch welche die Flüssigkeitsprobe aus dem Meßsystem abgeführt wird, eine weitere Lichtfalle 17 angeordnet.

Der wesentliche Unterschied zwischen den beiden Ausführungsformen der Figuren 2 und 3 besteht in folgendem:
(a) In der Ausführungsform nach Figur 2 ist eine gemeinsame Lichtquelle 18 zum Anregen von prompter und verzögerter Fluoreszenz vorgesehen, die natürlich der ersten Fluoreszenzmeß-Durchflußküvette 10 zugeordnet ist.
(b) Dagegen sind in der Ausführungsform nach Figur 3 zwei Lichtquellen vorgesehen, nämlich eine erste Lichtquelle 19 zum Anregen der prompten Fluoreszenz, die natürlich der ersten Fluoreszenzmeß-Durchflußküvette 10 zugeordnet ist, und eine zweite Lichtquelle 20 zum Anregen der verzögerten Fluoreszenz, die einer Fluoreszenzanregungs-Durchflußküvette 21 zugeordnet ist, welche in der Verbindungsleitung 12 zwischen der ersten Fluoreszenzmeß-Durchflußküvette 10 und der ersten Lichtfalle 13 vorgesehen ist.

Schließlich ist in Figur 4 eine Ausführungsform der Einrichtung gezeigt, welche eine gemeinsame Fluoreszenzmeß-Küvette 22 hat, die, beispielsweise über ein Durchflußventil 23, im diskontinuierlichen Durchfluß der Flüssigkeitsprobe betrieben werden kann und der eine Lichtquelle 24 zum Anregen der prompten und der verzögerten Fluoreszenz sowie ein Lichtdetektor 25 zum Messen der prompten und der verzögerten Fluoreszenz zugeordnet ist. Es sei hier darauf hingewiesen, daß die Lichtquelle 24 auch, obwohl in der Zeichnung nicht dargestellt, aus zwei gesonderten Lichtquellen bestehen kann, von denen die eine zum Anregen der prompten und die andere zum Anregen der verzögerten Fluoreszenz dient, und/oder daß der Lichtdetektor 25 einen ersten Lichtdetektor zum Messen der prompten und einen zweiten Lichtdetektor zum Messen der verzögerten Fluoreszenz umfassen kann.

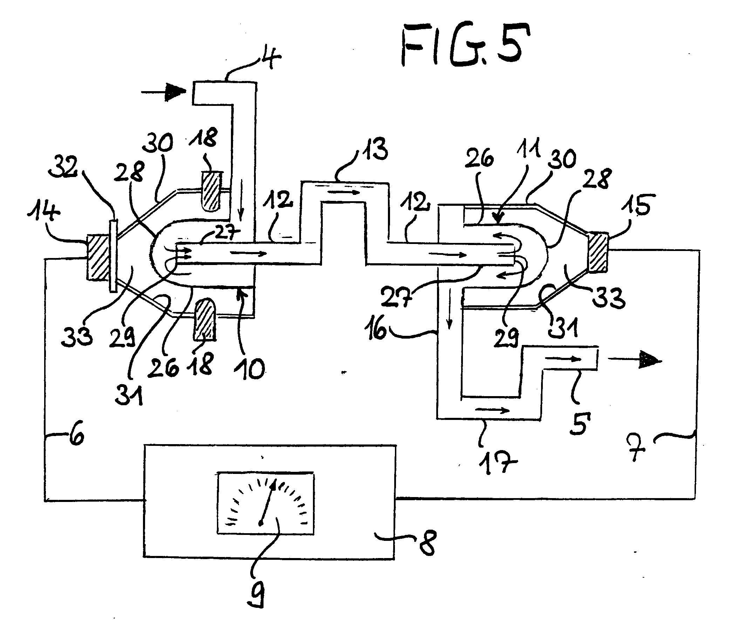

Die Figur 5 zeigt in näheren Einzelheiten eine Ausführungsform der Einrichtung zum Toxizitätsnachweis in Oberflächengewässern gemäß Figur 1 mit einer Anordnung der Fluoreszenzmeß-Durchflußküvetten 10 und 11, der Lichtquelle 18 und der Lichtdetektoren 14, 15 gemäß Figur 2. Hinsichtlich des grundsätzlichen Aufbaus wird daher auf die Erläuterungen der Figuren 1 und 2 verwiesen, und es werden nachstehend nur noch Besonderheiten, die über die Figuren 1 und 2 hinausgehen, erläutert:

Wie die Figur 5 zeigt, sind die Fluoreszenzmeß-Durchflußküvetten 10 und 11 vorliegend nach Art einer sogenannten Kühlfalle aufgebaut, die im wesentlichen aus einem zylindrischbecherförmigen äußeren Glasgefäß 26 und einem dazu konzentrischen inneren Glasrohr 27 kleineren Durchmessers besteht, dessen dem gerundeten Boden 28 des Glasgefäßes 26 im Abstand gegenüberliegendes freies Ende 29 im einen Falle als Eintrittsöffnung für die in das äußere Glasgefäß 26 zugeführte Probenflüssigkeit dient, wie links in Figur 5 dargestellt, während dieses freie Ende 29 im anderen Falle, nämlich rechts in Figur 5 als Austrittsöffnung für die in das äußere Glasgefäß 26 zuzuführende Flüssigkeitsprobe dient.

Die beiden Fluoreszenzmeß-Durchflußküvetten 10 und 11 sind gemäß Figur 5 konzentrisch von einem Küvettengehäuse 30 umgeben, das vorliegend aus einem zylindrischen und einem kegelstumpfförmigen Teil besteht und innen mit einer Verspiegelung 31 versehen ist, so daß die Fluoreszenzlichtstrahlung weitestgehend durch mehrfache Reflexion dem am verjüngten Kegelstumpfende gegenüber dem gerundeten Boden 28 vorgesehenen Lichtdetektor 14 bzw. 15 zugeführt wird.

Als Lichtquelle 18 zum Anregen von prompter und verzögerter Fluoreszenz sind Leuchtdioden vorgesehen, und vor dem Lichtdetektor 14 ist ein Filter 32 angeordnet, wobei hier darauf hingewiesen wird, daß erforderlichenfalls ein entsprechendes Lichtfilter auch vor dem Lichtdetektor 15 vorgesehen sein kann. Lichtfallen 13 und 17 sind als U-förmige Abschnitte der Verbindungsleitung 12 bzw. der Auslaßleitung 16 ausgebildet, können jedoch auch jede andere bekannte geeignete Form haben.

Der Ausgang 9 der Signalverknüpfungs- bzw. -korrelierungseinrichtung 8 wird vorliegend durch ein Anzeigeinstrument repräsentiert, das die Toxizität bzw. Nichttoxizität der gemessenen Flüssigkeitsprobe anzeigt. Selbstverständlich kann auch ein Schreiberausgang und/oder jeder gewünschte sonstige Ausgang, wie beispielsweise ein Drucker o.dgl. angeschlossen oder anstelle des Anzeigeinstruments vorgesehen sein.

Es seien nun anhand der vorstehend erläuterten Einrichtungen bevorzugte Verfahrensweisen zum Toxizitätsnachweis in Oberflächengewässern erläutert:

Zunächst kann für die Ausführungsformen der Einrichtungen nach den Figuren 2 und 4 die Anregungswellenlänge für die prompte und die verzögerte Fluoreszenz gleich sein und bei 660 bis 685 nm oder im Bereich um 440 nm liegen. Im ersteren Falle ist die Emissionswellenlänge der prompten Fluoreszenz bei etwa 730 nm und die der verzögerten Fluoreszenz breitbandig bei 685 nm. Im zweiten Falle liegt die Emissionswellenlänge von prompter und verzögerter Fluoreszenz bei 685 nm. Bei der Ausführungsform der Einrichtung nach Figur 3 können unterschiedliche Anregungswellenlängen für die prompte und der verzögerte Fluoreszenz gewählt werden, und zwar kann zusätzlich zu den vorstehend angegebenen Anregungswellenlängen für die verzögerte Fluoreszenz der Bereich der Anregungswellenlänge bei 700 bis 730 nm liegen. Diese Anregungswellenlängen gelten insbesondere für die Verwendung von Phytoplankton als toxizitätsempfindlicher Bioorganismus.

Bei dem kontinuierlichen Strömungssystem gemäß Figur 2 wird die Flüssigkeitsprobe in die Fluoreszenzmeß-Durchflußküvette 10 gepumpt, dort mittels der Lichtquelle 18 beleuchtet und die hierdurch angeregte prompte Fluoreszenz mittels des Lichtdetektors 14 gemessen. Durch die Lichtfalle 13, die auch aus mehreren parallelen Lichtfallen bestehen kann, wird die Flüssigkeitsprobe kontinuierlich in die Fluoreszenzmeß-Durchflußküvette 11 transportiert, in welcher die verzögerte Fluoreszenz mittels des Lichtdetektors 15 gemessen wird. Die aus der Fluoreszenzmeß-Durchflußküvette 11 austretende Flüssigkeitsprobe durchläuft die weitere Lichtfalle 17, bevor sie verworfen wird.

Bei dem in Figur 3 dargestellten kontinuierlichen Strömungssystem wird die prompte Fluoreszenz mittels der Lichtquelle 19 in der Fluoreszenzmeß-Durchflußküvette 10 angeregt und mittels des Lichtdetektors 14 gemessen. Die strömungsmäßig nachgeschaltete Fluoreszenzmeß-Küvette 21 dient zur Anregung der verzögerten Fluoreszenz mittels der Lichtquelle 20, und die strömungsmäßig nachfolgende Fluoreszenzmeß-Durchflußküvette 11 dient zur Messung der verzögerten Fluoreszenz mittels des Lichtdetektors 15.

Bei dem in Figur 4 gezeigten Meßsystem, das nach der Lichtpulsmethode in Verbindung mit einer einzigen Fluoreszenzmeß-Küvette 22 arbeitet, wird die Flüssigkeitsprobe disknotinuierlich in die Fluoreszenzmeß-Küvette 22 befördert und dort mittels der Lichtquelle 24 beleuchtet. Gleichzeitig wird die prompte Fluoreszenz mittels des Lichtdetektors 25 gemessen. Nach dem Ausschalten des Lichtes von der Lichtquelle 24 kann in der gleichen Fluoreszenzmeß-Küvette 22 die verzögerte Fluoreszenz mittels des gleichen Lichtdetektors 25 gemessen werden. Nach Abschluß dieses Meßzyklus kann eine neue Flüssigkeitsprobe in die Fluoreszenzmeß-Küvette durch Öffnen des Durchflußventils 23 eingeleitet werden.

Allgemein ist hinsichtlich aller Ausführungsformen der vorgeschlagenen Einrichtung zu bemerken, daß die Proben im kontinuierlichen Fließsystem oder durch Lichtimpulse mit starker Belichtung vorbehandelt werden können, um sie auf einen Standard-Lichtadaptionsstatus zu bringen. Weiterhin kann eine Heizung auf der Flüssigkeitsproben-Einlaufseite zur periodischen Wärmevorbehandlung, vorzugsweise über 50°C, der Proben vorgesehen sein, um so eine Nullpunktbestimmung der verzögerten Fluoreszenz sowie auch eine Reinigung der Küvetten vorzunehmen.

Bei der Einrichtung, die gemäß Figur 5 ausgebildet ist, wird die Flüssigkeitsprobe durch ein lichtdurchlässiges Rohr, beispielsweise ein Glasrohr, zur durchsichtigen, insbesondere aus Glas bestehenden, Fluoreszenzmeß-Durchflußküvette 10 gepumpt, in welche die Flüssigkeitsprobe tangential eintritt und spiralförmig zum Detektorende, nämlich dem Boden 28, der Fluoreszenzmeß-Durchflußküvette 10 geführt wird, während die Flüssigkeitsprobe von Leuchtdioden 18 beleuchtet wird. Die induzierte prompte Fluoreszenz wird aus dem äußeren, durchsichtigen Gefäß 26, das insbesondere ein Glasgefäß sein kann, in den spiegelnden Küvettenraum 33 zwischen dem äußeren Gefäß 26 und dem Küvettengehäuse 30 emittiert und gelangt durch das Lichtfilter 32 auf den Lichtdetektor 14, der beispielsweise ein Photomultiplier oder eine lichtempfindliche Diode sein kann und der die Intensität der prompten Fluoreszenz mißt. Die Flüssigkeitsprobe wird dann über das Glasrohr 27 und die Lichtfalle 13 an den detektornahen, durchsichtigen Boden 28 der Fluoreszenzmeß-Durchflußküvette 11 geleitet. Von dort wird die Flüssigkeitsprobe in Springbrunnenkonfiguration radial abgeleitet und durch die Lichtfalle 17 geführt und verworfen. Die in der Fluoreszenzmeß-Durchflußküvette 11 emittierte verzögerte Fluoreszenz wird in den verspiegelten Küvettenraum 33 emittiert und von dem Lichtdetektor 15, der auch beispielsweise ein Photomultiplier oder eine lichtempfindliche Diode sein kann, detektiert. Die Ausgangssignale der beiden Lichtdetektoren 14 und 15 werden der Signalverknüpfungs- bzw. -korrelierungseinrichtung 8 zugeführt, deren Ausgangsgröße am Ausgang 9 den Toxizitätsgrad der Flüssigkeitsprobe angibt.

Die Signalverknüpfungs- bzw. -korrelierungseinrichtung 8 kann ein Analog- oder Digitalrechner und im einfachsten Falle ein Quotientenbildungsgerät bzw. eine Divisionsschaltung sein.

Überall dort, wo Glas als Material von Bauteilen erwähnt ist, kann dieses auch durch Quarz oder ein anderes durchsichtiges Material, wie beispielsweise Kunststoff, ersetzt sein.

Die erwähnten Leuchtdioden, sogenannte LED's, können durch Filter spektral eingeschränkt werden. Die Leuchtdioden können durch Lampen ersetzt werden oder durch Lichtleiter, denen Licht von Lampen oder Leuchtdioden zugeführt wird.

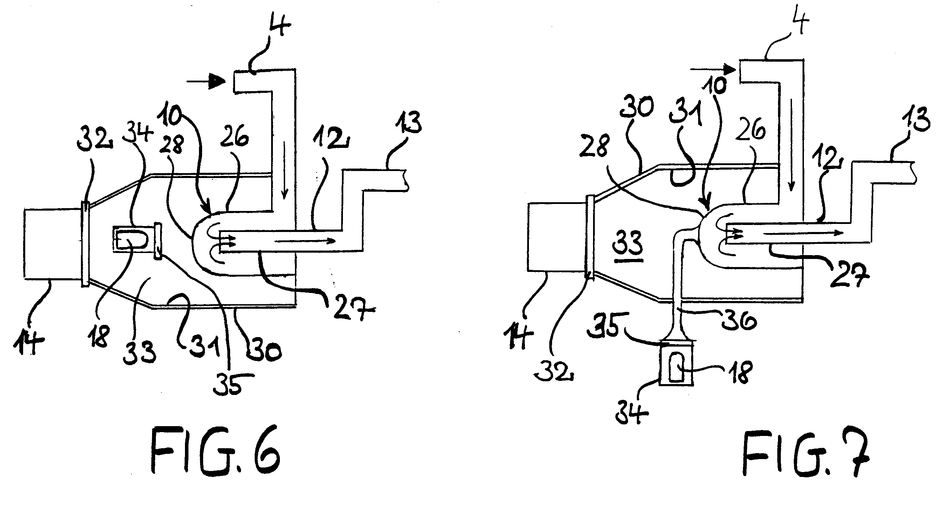

Während mehrere Leuchtdioden 18 in der Ausführungsform der Figur 5 in den Küvettenraum 33 hineinragen, ist in der Ausführungsform der Figur 6 eine einzige Leuchtdiode verhältnismäßig hoher Strahlungsleistung in einem kleinen Gehäuse 34 innerhalb des Küvettenraums 33 der Fluoreszenzmeß-Durchflußküvette 10 für die Messung der prompten Fluoreszenz vorgesehen. Das Gehäuse 34 ist zwischen dem Filter 32 und dem Boden 28 des Glasgefäßes 26 so angeordnet, daß sein Strahlungsaustrittsfenster, das von einem Filter 35 gebildet wird, dem Boden 28 zugewandt ist, wohingegen das Gehäuse 34 im übrigen lichtdicht ist, so daß der Detektor 14 weitestgehend von dem durch das Filter 35 austretenden Anregungslicht abgeschirmt ist. Das prompte Fluoreszenzlicht hingegen gelangt infolge der Verspiegelung 31 ohne weiteres zum Detektor 14.

Die Ausführungsform der Figur 7 unterscheidet sich von derjenigen der Figur 6 im wesentlichen dadurch, daß das kleine Gehäuse 34 außerhalb des Küvettengehäuses 30 angeordnet und das Licht der darin befindlichen Leuchtdiode nach Durchtritt durch das Filter 35 mittels eines Lichtleiters 36 zu der Küvette 10 zugeführt wird, und zwar so, daß es durch den Boden 28 in einer Richtung, die von dem Detektor 14 diametral weggerichtet ist, abgestrahlt wird, wodurch ebenfalls der Detektor weitestgehend vor dem Anregungslicht abgeschirmt wird.

Anstelle der Leuchtdioden 18 können natürlich auch Lampen oder andere Lichtquellen verwendet werden. Auch kann die Ausführungsform der Figur 7 so abgewandelt sein, daß der Lichtleiter 36 weggelassen wird und das Gehäuse 34 über ein Filter 35 direkt über einem Lichtschacht (nicht gezeigt) mit dem Boden 28 und/oder dem Glasgefäß 26 verbunden ist, was sich insbesondere bei relativ großer Lichtquelle empfiehlt, also wenn anstelle der Leuchtdiode 18 eine Lampe vorgesehen ist.

Hierbei stellen die beiden Filter 32 und 35 sicher, daß der Detektor 14 nicht zuviel Anregungslicht erhält, also die Messung des Emissionslichts der prompten Fluoreszenz weitestgehend von Störungen durch das Anregungslicht frei ist.

Bevorzugt sind die Filter 32 und 35 so gewählt, daß die Durchlaßkurve beider Filter 32 und 35 derart ist, daß die Menge an Licht, die durch die beiden Filter in Hintereinanderanordnung bei 3 σ (σ = Halbwertsbreite) noch hindurchtritt, kleiner als 10⁻³, vorzugsweise bei 6 σ kleiner als 10⁻⁸, der Intensität des auf die Filter auftreffenden anregenden Lichts an dieser spektralen Stelle ist. Hierbei ist bevorzugt die Anregungswellenlänge etwa 665 nm und die Emissionswellenlänge der prompten Fluoreszenz etwa 730 nm. Die obige Bedingung gilt aber auch für andere Δλ-Werte, wobei Δλ die Differenz zwischen der Wellenlänge der maximalen Emissionslichtdurchlässigkeit der prompten Fluoreszenz und der Wellenlänge der maximalen Anregungslichtdurchlässigkeit ist.

## Patentansprüche

1. Verfahren zum Toxizitätsnachweis in Oberflächengewässern sowie in Trink- und Brauchwasser, bei dem die Fluoreszenz einer Wasserprobe gemessen wird, dadurch **gekennzeichnet,** daß die Korrelation zwischen der prompten Fluoreszenz und der verzögerten Fluoreszenz einer Wasserprobe, die einen toxizitätsempfindlichen Bioorganismus enthält, bestimmt wird und mit der entsprechenden Korrelation verglichen wird, die an einer nichtvergifteten Probe bestimmt worden ist.

2. Verfahren nach Anspruch 1, dadurch **gekennzeichnet,** daß die Korrelation innerhalb eines vorbestimmten Zeitfensters ab dem Ende des Einwirkens des Anregungslichts für die verzögerte Fluoreszenz auf die Probe gemessen wird, wobei dieses Zeitfenster vorzugsweise eine Breite von 0,1 bis 10 Sekunden hat und bevorzugt in einem Bereich von 0,5 bis 500 Sekunden nach dem Ende des Einwirkens des Anregungslichts für die verzögerte Fluoreszenz liegt.

3. Verfahren nach Anspruch 1 oder 2, dadurch **gekennzeichnet,** daß der toxizitätsempfindliche Bioorganismus ein Plankton, insbesondere ein Phytoplankton, ist oder daß als toxizitätsempfindliche Bioorganismen Bakterien oder Dinoflagellanten verwendet werden.

4. Verfahren nach Anspruch 1, 2 oder 3, dadurch **gekennzeichnet,** daß die prompte und verzögerte Fluoreszenz mittels Licht der gleichen Wellenlänge angeregt werden.

5. Verfahren nach Anspruch 4, dadurch **gekennzeichnet,** daß zum Anregen der prompten und verzögerten Fluoreszenz Licht im Wellenlängenbereich um 440 nm verwendet wird.

6. Verfahren nach Anspruch 4, dadurch **gekennzeichnet,** daß zum Anregen der prompten und verzögerten Fluoreszenz Licht im Wellenlängenbereich um 660 nm bis 685 nm verwendet wird.

7. Verfahren nach Anspruch 1, 2 oder 3, dadurch **gekennzeichnet,** daß die prompte und verzögerte Fluoreszenz mittels Licht unterschiedlicher Wellenlängen angeregt werden.

8. Verfahren nach Anspruch 7, dadurch **gekennzeichnet,** daß die prompte Fluoreszenz mittels Licht im Wellenlängenbereich um 440 nm und/oder um 660 bis 685 nm und die verzögerte Fluoreszenz mittels Licht im Wellenlängenbereich um 700 bis 730 nm angeregt werden.

9. Verfahren nach einem der Ansprüche 1 bis 8, dadurch **gekennzeichnet,** daß die prompte und verzögerte Fluoreszenz im gleichen Wellenlängenbereich gemessen werden.

10. Verfahren nach den Ansprüchen 5 und 9 oder nach den Ansprüchen 8 und 9, dadurch **gekennzeichnet,** daß die prompte und verzögerte Fluoreszenz im Wellenlängenbereich um 685 nm gemessen werden.

11. Verfahren nach einem der Ansprüche 1 bis 8, dadurch **gekennzeichnet,** daß die prompte und verzögerte Fluoreszenz in unterschiedlichen Wellenlängenbereichen gemessen werden.

12. Verfahren nach den Ansprüchen 6 und 11 oder nach den Ansprüchen 8 und 11, dadurch **gekennzeichnet,** daß die prompte Fluoreszenz im Wellenlängenbereich um 730 nm und die verzögerte Fluoreszenz im Wellenlängenbereich um 685 nm gemessen werden.

13. Einrichtung zum Toxizitätsnachweis in Oberflächengewässern, umfassend eine Fluoreszenzmeßeinrichtung (1) für Fluoreszenzmessungen an Flüssigkeiten, dadurch **gekennzeichnet,** daß die Fluoreszenzmeßeinrichtung (1) eine Kombination aus einer ersten Fluoreszenzmeßeinrichtung (2) zum Messen der prompten Fluoreszenz und einer zweiten Fluoreszenzmeßeinrichtung (3) zum Messen der verzögerten Fluoreszenz umfaßt und den Meßsignalausgängen (6,7) der beiden Fluoreszenzmeßeinrichtungen (2,3) eine gemeinsame Signalverknüpfungs- bzw. -korrelierungseinrichtung (8) zum Miteinanderverknüpfen bzw. Korrelieren ihrer Ausgangssignale nachgeschaltet ist.

14. Einrichtung nach Anspruch 13, **gekennzeichnet** durch zwei strömungsmäßig hintereinandergeschaltete Fluoreszenzmeß-Durchflußküvetten (10,11), deren Verbindungsleitung (12) eine Lichtfalle (13) enthält und von denen der in Durchflußrichtung ersten Fluoreszenzmeß-Durchflußküvette (10) ein erster Lichtdetektor (14) zum Messen der prompten Fluoreszenz und der in Durchflußrichtung zweiten Fluoreszenzmeß-Durchflußküvette (11) ein zweiter Lichtdetektor (15) zum Messen der verzögerten Fluoreszenz zugeordnet ist.

15. Einrichtung nach Anspruch 14, dadurch **gekennzeichnet,** daß der ersten Fluoreszenzmeß-Durchflußküvette (10) eine Lichtquelle (18) zum Anregen von prompter und verzögerter Fluoreszenz zugeordnet ist (Figur 2).

16. Einrichtung nach Anspruch 14, dadurch **gekennzeichnet,** daß der ersten Fluoreszenzmeß-Durchflußküvette (10) eine erste Lichtquelle (19) zum Anregen von prompter Fluoreszenz zugeordnet ist und daß strömungsmäßig zwischen die erste und zweite Fluoreszenzmeß-Durchflußküvette (10,11) eine Fluoreszenzanregungs-Durchflußküvette (21) geschaltet ist, der eine zweite Lichtquelle (20) zum Anregen von verzögerter Fluoreszenz zugeordnet ist (Figur 3).

17. Einrichtung nach Anspruch 16, dadurch **gekennzeichnet,** daß die Lichtfalle (13) in der Verbindungsleitung (12) zwischen der Fluoreszenzanregungs-Durchflußküvette (21) und der zweiten Fluoreszenzmeß-Durchflußküvette (11) vorgesehen ist (Figur 3).

18. Einrichtung nach Anspruch 13, **gekennzeichnet** durch eine gemeinsame Fluoreszenzmeß-Küvette (22) für die Messung der prompten und verzögerten Fluoreszenz (Figur 4).

19. Einrichtung nach einem der Ansprüche 14 bis 17, dadurch **gekennzeichnet,** daß in der Auslaßleitung (16) der zweiten Fluoreszenzmeß-Durchflußküvette (11) eine weitere Lichtfalle (17) vorgesehen ist.

20. Einrichtung nach einem der Ansprüche 13 bis 19, dadurch **gekennzeichnet,** daß die Signalverknüpfungs- bzw. -korrelierungseinrichtung (8) ein Analogoder Digitalrechner ist.

21. Einrichtung nach einem der Ansprüche 13 bis 19, dadurch **gekennzeichnet,** daß die Signalverknüpfungs- bzw. -korrelierungseinrichtung (8) ein Quotientenbildungsgerät ist.

## Claims

1. A method of detecting toxicity in surface waters and in drinking water and industrial water, wherein the fluorescence of a water sample is measured, characterised in that the correlation between the prompt fluorescence and the delayed fluorescence of a water sample containing a toxicity-sensitive biological organism is determined and is compared with the corresponding correlation which has been determined in the case of a non-toxic sample.

2. A method as claimed in Claim 1, characterised in that the correlation is measured within a predetermined time window from the end of the influence upon the sample of the exciting light for the delayed fluorescence, where said time window preferably has a breadth of 0.1 to 10 seconds and preferably is located in a range of 0.5 to 500 seconds following the end of the influence of the exciting light for the delayed fluorescence.

3. A method as claimed in Claim 1 or 2, characterised in that the toxicity-sensitive biological organism is plankton, in particular phytoplankton or in that bacteria or dinoflagellates are used as toxicity-sensitive biological organisms.

4. A method as claimed in Claim 1, 2 or 3, characterised in that the prompt fluorescence and delayed fluorescence are excited by means of light of the same wave length.

5. A method as claimed in Claim 4, characterised in that light in the wave length range around 440 nm is used to excite the prompt fluorescence and the delayed fluorescence.

6. A method as claimed in Claim 4, characterised in that light in the wave length range around 660 nm to 685 nm is used to excite the prompt fluorescence and the delayed fluorescence.

7. A method as claimed in Claim 1, 2 or 3, characterised in that the prompt fluorescence and delayed fluorescence are excited by means of light of different wave lengths.

8. A method as claimed in Claim 7, characterised in that the prompt fluorescence is excited by means of light in the wave length range around 440 nm and/or around 660 to 685 nm, and the delayed fluorescence is excited by means of light in the wave length range around 700 to 730 nm.

9. A method as claimed in one of Claims 1 to 8, characterised in that the prompt fluorescence and the delayed fluorescence are measured in the same wave length range.

10. A method as claimed in Claims 5 and 9 or in Claims 8 and 9, characterised in that the prompt fluorescence and the delayed fluorescence are measured in the wave length range around 685 nm.

11. A method as claimed in one of Claims 1 to 8, characterised in that the prompt fluorescence and the delayed fluorescence are measured in different wave length ranges.

12. A method as claimed in Claims 6 and 11 or in Claims 8 and 11, characterised in that the prompt fluorescence is measured in the wave length range around 730 nm and the delayed fluorescence is measured in the wave length range around 685 nm.

13. A device for detecting toxicity in surface waters, comprising a fluorescence measuring device (1) for fluorescence measurements of liquids, characterised in that the fluorescence measuring device (1) comprises a combination of a first fluorescence measuring device (2) for the measurement of the prompt fluorescence and a second fluorescence measuring device (3) for the measurement of the delayed fluorescence, and the measuring signal outputs (6, 7) of the two fluorescence measuring devices (2, 3) are connected at their output end to a common signal combining- or correlating device (8) for the combining or correlating of their output signals.

14. A device as claimed in Claim 13, characterised by two fluorescence measuring flow cells (10, 11) which are connected in series in terms of the flow, and the connecting line (12) between which contains a light trap (13), and of which the first fluorescence measuring flow cell (10) considered in the flow direction is assigned a first light detector (14) for the measurement of the prompt fluorescence and the second fluorescence measuring flow cell (11) considered in the flow direction is assigned a second light detector (15) for the measurement of the delayed fluorescence.

15. A device as claimed in Claim 14, characterised in that the first fluorescence measuring flow cell (10) is assigned a light source (18) for the excitation of prompt fluorescence and delayed fluorescence (Figure 2).

16. A device as claimed in Claim 14, characterised in that the first fluorescence measuring flow cell (10) is assigned a first light source (19) for the excitation of prompt fluorescence, and that in the flow path between the first and second fluorescence measuring flow cells (10, 11) is arranged a fluorescence excitation flow cell (21) which is assigned a second light source (20) for the excitation of delayed fluorescence (Figure 3).

17. A device as claimed in Claim 16, characterised in that the light trap (13) is arranged in the connecting line (12) between the fluorescence excitation flow cell (21) and the second fluorescence measuring flow cell (11) (Figure 3).

18. A device as claimed in Claim 13, characterised by a common fluorescence measuring cell (22) for the measurement of the prompt fluorescence and the delayed fluorescence (Figure 4).

19. A device as claimed in one of Claims 14 to 17, characterised in that a further light trap (17) is arranged in the outlet line (16) of the second fluorescence measuring flow cell (11).

20. A device as claimed in one of Claims 13 to 19, characterised in that the signal combining- or correlating device (8) is an analogue- or digital computer.

21. A device as claimed in one of Claims 13 to 19, characterised in that the signal combining- or correlating device (8) is a quotient forming device.

## Revendications

1. Procédé pour détecter la toxicité d'eaux de ruissellement ainsi que d'eaux potables ou non potables, selon lequel on mesure la fluorescence d'un échantillon d'eau, **caractérisé** en ce qu'on détermine la corrélation entre la fluorescence instantanée et la fluorescence retardée d'un échantillon d'eau contenant un bioorganisme sensible à la toxicité, et on la compare à la corrélation correspondante qui a été déterminée sur un échantillon non pollué.

2. Procédé selon la revendication 1, **caractérisé** en ce que la corrélation est mesurée à l'intérieur d'une fenêtre de temps prédéterminé à partir de la fin de l'action sur l'échantillon de la lumière provoquant la fluorescence retardée, cette fenêtre de temps possédant de préférence une largeur de 0,1 à 10 secondes et se trouvant de préférence dans une plage de 0,5 à 500 secondes après la fin de l'action de la lumière provoquant la fluorescence retardée.

3. Procédé selon la revendication 1 ou 2, **caractérisé** en ce que le bioorganisme sensible à la toxicité est un plancton, notamment un phytoplancton, ou en ce qu'on utilise comme bioorganismes sensibles à la toxicité des bactéries ou des dinoflagellants.

4. Procédé selon la revendication 1, 2 ou 3, **caractérisé** en ce que la fluorescence instantanée et la fluorescence retardée sont provoquées par de la lumière de même longueur d'onde.

5. Procédé selon la revendication 4, **caractérisé** en ce qu'on utilise pour provoquer la fluorescence instantanée et la fluorescence retardée de la lumière d'environ 440 nm de longueur d'onde.

6. Procédé selon la revendication 4, **caractérisé** en ce qu'on utilise pour provoquer la fluorescence instantanée et la fluorescence retardée de la lumière d'environ 660 à 685 nm de longueur d'onde.

7. Procédé selon la revendication 1, 2 ou 3, **caractérisé** en ce que la fluorescence instantanée et la fluorescence retardée sont provoquées par de la lumière de longueurs d'onde différentes.

8. Procédé selon la revendication 7, **caractérisé** en ce que la fluorescence instantanée est provoquée par de la lumière d'environ 440 nm et/ou d'environ 660 à 685 nm de longueur d'onde, et la fluorescence retardée est provoquée par de la lumière d'environ 700 à 730 nm de longueur d'onde.

9. Procédé selon l'une quelconque des revendications 1 à 8, **caractérisé** en ce que la fluorescence instantanée et la fluorescence retardée sont mesurées dans la même plage de longueur d'onde.

10. Procédé selon les revendication 5 et 9 ou selon les revendications 8 et 9, **caractérisé** en ce que la fluorescence instantanée et la fluorescence retardée sont mesurées dans la plage de longueur d'onde d'environ 685 nm.

11. Procédé selon l'une quelconque des revendications 1 à 8, **caractérisé** en ce que la fluorescence instantanée et la fluorescence retardée sont mesurées dans des plages différentes de longueur d'onde.

12. Procédé selon les revendications 6 et 11 ou selon les revendications 8 et 11, **caractérisé** en ce que la fluorescence instantanée est mesurée dans la plage de longueur d'onde d'environ 730 nm, et la fluorescence retardée est mesurée dans la plage de longueur d'onde d'environ 685 nm.

13. Dispositif pour détecter la toxicité d'eaux de surface, comprenant un dispositif de mesure de fluorescence (1) pour des mesures de fluorescence sur des liquides, **caractérisé** en ce que le dispositif de mesure de fluorescence (1) comprend une combinaison d'un premier dispositif de mesure de fluorescence (2) pour mesurer la fluorescence instantanée et d'un second dispositif de mesure de fluorescence (3) pour mesurer la fluorescence retardée, et les sorties de signaux de mesure (6, 7) des deux dispositifs de mesure de fluorescence (2, 3) sont reliés à un dispositif commun (8) pour combiner ou corréler les signaux, afin de combiner ou corréler leurs signaux de sortie.

14. Dispositif selon la revendication 13, **caractérisé** par deux cuvettes de passage pour mesurer la fluorescence (10, 11), qui se succèdent en matière d'écoulement et dont la conduite de jonction (12) contient un piège de lumière (13), un premier détecteur de lumière (14) étant associé à la première cuvette (10), dans la direction de circulation (10), pour mesurer la fluorescence instantanée, tandis qu'un second détecteur de lumière (15) est associé à la seconde cuvette (11), dans la direction de circulation, pour mesurer la fluorescence retardée.

15. Dispositif selon la revendication (14), **caractérisé** en ce qu'une source de lumière (18) est associée à la première cuvette de passage (10) pour mesurer la fluorescence, afin de provoquer la fluorescence instantanée et la fluorescence retardée (figure 2).

16. Dispositif selon la revendication 14, **caractérisé** en ce qu'une première source de lumière (19) est associée à la première cuvette de passage (10) pour mesurer la fluorescence, afin de provoquer la fluorescence instantanée, et en ce qu'une cuvette de passage (21) pour provoquer la fluorescence est reliée, en matière d'écoulement, entre la première et la seconde cuvettes de passage (10, 11) pour mesurer la fluorescence, une seconde source de lumière (20) étant associée à la cuvette de passage (21) afin de provoquer la fluorescence retardée (figure 3).

17. Dispositif selon la revendication 16, **caractérisé** en ce que le piège de lumière (13) est prévu dans la conduite de jonction (12) entre la cuvette de passage (21) pour provoquer la fluorescence et la seconde cuvette de passage (11) pour mesurer la fluorescence (figure 3).

18. Dispositif selon la revendication 13, **caractérisé** par une cuvette commune (22) pour mesurer la fluorescence, pour la mesure de la fluorescence instantanée et de la fluorescence retardée (figure 4).

19. Dispositif selon l'une quelconque des revendications 14 à 17, **caractérisé** en ce qu'un piège de lumière supplémentaire (17) est prévu dans la conduite de sortie (16) de la seconde cuvette de passage (11) pour mesurer la fluorescence.

20. Dispositif selon l'une quelconque des revendications 13 à 19, **caractérisé** en ce que le dispositif (8) pour combiner ou corréler les signaux est un calculateur analogique ou numérique.

21. Dispositif selon l'une quelconque des revendications 13 à 19, **caractérisé** en ce que le dispositif (8) pour combiner ou corréler les signaux est un formateur de quotient.
